# Europäisches Patentamt

# European Patent Office

# Office européen des brevets

(11) Veröffentlichungsnummer: **0 243 681 B1**

(12) **EUROPÄISCHE PATENTSCHRIFT**

(45) Veröffentlichungstag der Patentschrift:
13.06.90

(51) Int. Cl.⁵: **G01N 27/04**, G01N 33/38

(21) Anmeldenummer: 87104446.7

(22) Anmeldetag: 25.03.87

(54) Verfahren und Einrichtung zum Erfassen des Abbindevorganges in einem anorganischen, wässrigen Bindemittelsystem.

(30) Priorität: 29.04.86 DE 3614468

(43) Veröffentlichungstag der Anmeldung:
04.11.87 Patentblatt 87/45

(45) Bekanntmachung des Hinweises auf die Patenterteilung:
13.06.90 Patentblatt 90/24

(84) Benannte Vertragsstaaten:
CH DE ES LI

(56) Entgegenhaltungen:
DE-C- 430 768
US-A- 3 965 414
US-A- 4 120 166
US-A- 4 524 319

PATENT ABSTRACTS OF JAPAN, Band 6,
Nr. 18 (P-100)[896], 2. Februar 1982 & JP-A-56 141 548

(73) Patentinhaber: Siemens Aktiengesellschaft,
Wittelsbacherplatz 2, D-8000 München 2(DE)

(72) Erfinder: Schmitt, Wolfgang, Dreieichstrasse 1,
D-6078 Neu Isenburg(DE)
Erfinder: Bege, Dietmar, Schwalbenweg 10,
D-8520 Erlangen(DE)

## Beschreibung

Die Erfindung betrifft ein Verfahren zum Erfassen des Abbindevorganges in einem anorganischen, wäßrigen Bindemittelsystem gemäß dem Oberbegriff des Anspruchs 1 und eine Einrichtung gemäß dem Oberbegriff des Anspruchs 3.

Ein derartiges Bindemittelsystem besteht aus einem anorganischen Bindemittel und Wasser oder einer wäßrigen Salzlösung. Ein geeignetes anorganisches Bindemittel ist Zement. Eine Mischung aus Bindemittel und Wasser ist zunächst gießfähig und bindet durch eine chemische Reaktion ab.

Bindemittelsysteme aus Zement und Wasser werden eingesetzt, um Abfälle, insbesondere radioaktiv kontaminierte Abfälle in Beton einzubinden. Dabei wird der Abbindevorgang meßtechnisch erfaßt.

Auch in der Betonchemie werden Laboruntersuchungen zum Verhalten von Beton beim Abbinden durchgeführt.

Bekannte Verfahren zum Erfassen des Abbindevorganges sind das Nadelpenetrationsverfahren und das kalorimetrische Verfahren. Beim Nadelpenetrationsverfahren wird die Eindringtiefe einer mit einem Gewicht belasteten Nadel in das Bindemittelsystem ermittelt. Eine Abnahme der Eindringtiefe weist auf den Abbindevorgang hin.

Das kalorimetrische Verfahren sieht vor, daß der Temperaturverlauf einer thermisch isoliert aufgestellten Probe registriert wird. Da beim Abbinden Wärmeenergie frei wird, weist eine Temperaturerhöhung auf den Abbindevorgang hin.

Mit den bekannten Verfahren ist es oft nicht möglich, den Abbindevorgang, insbesondere bei langsamem Ablauf der chemischen Reaktion im Bindemittelsystem, zu erfassen. Bei einer Reaktion, die mehrere Tage in Anspruch nimmt, ist die Temperaturerhöhung so gering, daß das kalorimetrische Verfahren versagt. Das Nadelpenetrationsverfahren gibt nur Hinweise auf den Abbindevorgang in Oberflächenschichten. Erkenntnisse über den Abbindevorgang im Inneren eines Körpers sind mit diesem Verfahren unmöglich.

Aus US-A 4 524 319 ist ein Verfahren bzw. eine Einrcihtung gemäß dem Oberbegriff des Anspruchs 1 bzw. 3 bekannt.

Der Erfindung liegt die Aufgabe zugrunde, ein Verfahren und eine Einrichtung zum Erfassen des Abbindevorganges in einem anorganischen, wäßrigen Bindemittelsystem zu entwickeln, das die chemische Reaktion des Abbindens meßtechnisch mit großer Genauigkeit erfaßt und eine genaue Bestimmung des Abbindezeitpunkts ermöglicht.

Die Aufgabe wird erfindungsgemäß durch die Merkmale der Ansprüche 1 bzw. 3 gelöst.

Die chemische Reaktion des Abbindens im Bindemittelsystem führt zu einer Veränderung der Konzentration von leitfähigen Lösungen. Die Leitfähigkeit im Bindemittelsystem ist also mit dem Ablauf der chemischen Reaktion korreliert. Eine meßtechnisch genaue Überwachung der Leitfähigkeitsänderungen wird dadurch erzielt, daß statt der Leitfähigkeit, die erste Ableitung der Leitfähigkeit nach der Zeit, also deren Änderungsgeschwindigkeit überwacht wird. Schon eine geringe, aber schnelle Änderung der Leitfähigkeit bewirkt eine im Vergleich dazu große Änderung ihrer ersten Ableitung.

Da neben dem Abbindevorgang auch Temperaturschwankungen die Leitfähigkeit beeinflussen, wird im Bindemittelsystem beispielsweise auch die Temperatur kontinuierlich gemessen. Mit den gemessenen Temperaturwerten wird die Temperaturabhängigkeit der elektrischen Leitfähigkeit eliminiert. Es wird dann der korrigierte, temperaturunabhängige Verlauf der elektrischen Leitfähigkeit nach der Zeit differenziert.

Mit dem erfindungsgemäßen Verfahren wird der Vorteil erzielt, daß ständig exakte Informationen über die chemische Reaktion im Bindemittelsystem vorliegen. Der Zeitpunkt des Abbindens ist erfindungsgemäß, insbesondere bei Berücksichtigung des Temperaturverlaufes exakt bestimmbar.

Eine Einrichtung zur Durchführung des Verfahrens weist einen Leitfähigkeitsmesser auf, der in einem Bindemittelsystem, dessen Abbindevorgang überwacht werden soll, angeordnet ist. Der Leitfähigkeitsmesser weist beispielsweise zwei Elektroden auf, die in das Bindemittelsystem eingebracht werden. Statt dessen ist auch eine elektrodenlose Leitfähigkeitsmeßzelle einsetzbar. Der Ausgang des Leitfähigkeitsmessers ist mit einem Differenzierglied verbunden, in dem die erste Ableitung des gemessenen Leitfähigkeitsverlaufes nach der Zeit gegebildet wird. Dem Differenzierglied ist ein Anzeigegerät nachgeschaltet. Dort wird stets der momentane, differenzierte Leitfähigkeitswert angezeigt.

Zum Erfassen des Temperaturverlaufes im Bindemittelsystem ist dort beispielsweise ein Temperatursensor angeordnet. Der Leitfähigkeitsmesser und der Temperatursensor stehen mit einer Auswerteeinheit in Verbindung. Dort werden die Leitfähigkeitswerte hinsichtlich ihrer Temperaturabhängigkeit korrigiert. Der Ausgang der Auswerteeinheit ist mit dem Differenzierglied verbunden. Am Anzeigegerät wird dann stets der momentane korrigierte und differenzierte Leitfähigkeiswert angezeigt.

Dieser Wert ist ein gutes Maß für den Ablauf der chemischen Reaktion im Bindemittelsystem.

Mit der Erfindung wird der Vorteil erzielt, daß der chemische Vorgang des Abbindens von sich langsam verfestigenden Mischungen aus anorganischen Bindemitteln und Wasser oder wäßrigen Salzlösungen meßtechnisch mit großer Genauigkeit erfaßt wird. Dadurch läßt sich der Zeitpunkt des Abbindens, beispielsweise beim Einbinden von radioaktiv kontaminierten Abfällen in Beton genau bestimmen.

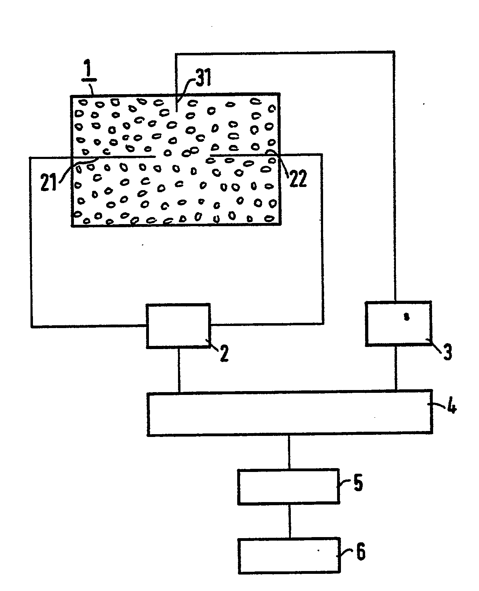

Die Erfindung wird anhand der Zeichnung näher erläutert:

Die Zeichnung zeigt eine Einrichtung zum Erfassen des Abbindevorganges in einem anorganischen, wäßrigen Bindemittelsystem.

Ein Bindemittelsystem 1 besteht aus einer Mischung aus anorganischen Bindemitteln und Wasser. Die chemische Reaktion dieses Systems wird überwacht, um den Zeitpunkt des Abbindens zu ermitteln. Dazu sind im Bindemittelsystem 1 zwei Elektroden 21 und 22 eines Leitfähigkeitsmessers 2 einander gegenüberliegend angeordnet. Mit dem Leitfähigkeitsmesser 2 wird der kontinuierliche Verlauf der elektrischen Leitfähigkeit im Bindemittelsystem 1 bestimmt. Außerdem ist im Bindemittelsystem 1 ein Fühler 31 eines Temperatursensors 3 angeordnet, der den Temperaturverlauf bestimmt. Der Leitfähigkeitsmesser 2 und der Temperatursensor 3 stehen mit einer Auswerteeinheit 4 in Verbindung. Dort werden die gleichzeitig einlaufenden Leitfähigkeits- und Temperaturwerte verknüpft. Dadurch erhält man den temperaturunabhängigen zeitlichen Verlauf der elektrischen Leitfähigkeit im Bindemittelsystem 1. Der Ausgang der Auswerteeinheit 4 ist mit einem Differenzierglied 5 verbunden. Dort wird für den Leitfähigkeitsverlauf die erste Ableitung nach der Zeit gebildet. Dadurch wird die Änderungsgeschwindigkeit der Leitfähigkeit bestimmt. Diese Größe ermöglicht es, den Zeitpunkt des Abbindens genau zu erfassen. Der Ausgang des Differenzergliedes 5 ist mit einem Anzeigegerät 6 verbunden.

## Patentansprüche

1. Verfahren zum Erfassen des Abbindevorganges in einem anorganischen, wäßrigen Bindemittelsystem (1), indem die elektrische Leitfähigkeit kontinuierlich gemessen wird, dadurch gekennzeichnet, daß der Verlauf der elektrischen Leitfähigkeit nach der Zeit differenziert wird und daß der Verlauf der ersten Ableitung der Leitfähigkeit überwacht wird.

2. Verfahren nach Anspruch 1, dadurch gekennzeichnet, daß im Bindemittelsystem (1) die Temperatur kontinuierlich gemessen wird und daß mit den gemessenen Temperaturwerten die Temperaturabhängigkeit der elektrischen Leitfähigkeit eliminiert und der korrigierte, temperaturunabhängige Verlauf der elektrischen Leitfähigkeit nach der Zeit differenziert wird.

3. Einrichtung zum Erfassen des Abbindevorganges in einem anorganischen, wäßrigen Bindemittelsystem (1) und zur Durchführung des Verfahrens nach Anspruch 1, wobei mit dem zu überwachenden Bindemittelsystem (1) ein Leitfähigkeitsmesser (2) verbunden ist, dadurch gekennzeichnet, daß Ausgang des Leitfähigkeitsmessers (2) mit einem Differenzierglied (5) verbunden ist, in dem die erste Ableitung nach der Zeit des Leitfähigkeitsverlaufes gebildet wird, und daß der Ausgang des Differenzergliedes (5) mit einem Anzeigegerät (6) für den differenzierten Leitfähigkeitsverlauf im Bindemittelsystem (1) verbunden ist.

4. Einrichtung nach Anspruch 3 zur Durchführung des Verfahrens nach den Ansprüchen 1 und 2, dadurch gekennzeichnet, daß mit dem zu überwachenden Bindemittelsystem (1) ein Temperatursensor (3) verbunden ist, daß der Leitfähigkeitsmesser (2) und der Temperatursensor (3) mit einer Auswerteeinheit (4) verbunden sind, in der die Leitfähigkeitswerte hinsichtlich ihrer Temperaturabhängigkeit korrigiert werden, daß der Ausgang der Auswerteeinheit (4), an dem ein Signal für den temperaturunabhängigen Leitfähigkeitsverlauf im Bindemittelsystem (1) ansteht, mit dem Differenzierglied (5) verbunden ist.

## Claims

1. Method for determining the setting process in an inorganic aqueous binder system (1) in which the electrical conductivity is continuously measured, characterised in that the variation of the electrical conductivity is differentiated with respect to time and in that the variation of the first derivative of the conductivity is monitored.

2. Method according to claim 1, characterised in that the temperature in the binder system (1) is continuously measured and in that the temperature dependence of the electrical conductivity is eliminated by means of the measured temperature values, and the corrected, temperature-independent variation of the electrical conductivity is differentiated with respect to time.

3. Apparatus for determining the setting process in an inorganic aqueous binder system (1) for carrying out the process according to claim 1, wherein a conductivity meter (2) is connected to the binder system (1) to be monitored, characterised in that the output of the conductivity meter (2) is connected to a differentiating element (5) in which the first derivative with respect to time of the conductivity variation is taken, and in that the output of the differentiating element (5) is connected to an indicating device (6) for the differentiated conductivity variation in the binder system (1).

4. Apparatus according to claim 3 for carrying out the method according to claims 1 and 2, characterised in that a temperature sensor (3) is connected to the binder system (1) to be monitored, in that the conductivity meter (2) and the temperature sensor (3) are connected to an analysing unit (4), in which the conductivity values are corrected with regard to their temperature dependence, in that the output of the analysing unit (4), at which output there is a signal for the temperature-independent conductivity variation in the binder system, is connected to the differentiating element (5).

## Revendications

1. Procédé de détection du processus de prise d'un système aqueux de liant minéral (1), dans lequel on mesure en continu la conductibilité électrique, caractérisé, en ce qu'il consiste à différencier en fonction du temps la variation de la conductibilité électrique et à surveiller la variation de la dérivé première de la conductibilité.

2. Procédé suivant la revendication 1, caractérisé, en ce qu'il consiste à mesurer en continu la température du système liant (1) et à éliminer, par les valeurs de température qui sont mesurées, la variation de la conductibilité électrique en fonction de la température et à différencier en fonction du temps la variation, corrigée et indépendante de la température, de la conductibilité électrique.

3. Dispositif de détection du processus de prise d'un système aqueux de liant minéral (1) et de mise en œuvre du procédé suivant la revendication 1, dans lequel un dispositif de mesure de la conductibilité (2) est relié au système de liant (1) à surveiller, caractérisé, en ce que la sortie du dispositif de mesure de la conductibilité (2) est reliée à un circuit de différentiation (5), dans lequel est formée la dérivée première en fonction du temps de la variation de la conductibilité et en ce que la sortie du circuit de différentiation (5) est reliée à un appareil d'affichage (6) de la variation différentielle de la conductibilité du système liant (1).

4. Dispositif suivant la revendication 3 et destiné à la mise en œuvre du procédé suivant les revendications 1 et 2, caractérisé, en ce qu'au système liant (1) à surveiller est reliée une sonde de température (3), en ce que le dispositif de mesure de la conductibilité (2) et la sonde de température (3) sont reliés à une unité d'exploitation (4), dans laquelle les valeurs de la conductibilité sont corrigées de leurs variations en fonction de la température, en ce que la sortie de l'unité d'exploitation (4), sur laquelle est appliqué un signal représentatif de la variation indépendamment de la température de la conductibilité du système liant (1), est reliée au circuit de différentiation (5).